(19) Europäisches Patentamt
European Patent Office
Office européen des brevets

(11) Publication number : **0 469 800 A2**

(12) **EUROPEAN PATENT APPLICATION**

(21) Application number : **91306856.5**

(22) Date of filing : **26.07.91**

(51) Int. Cl.[5] : **C09K 19/42,** C09K 19/30, C09K 19/12, C09K 19/46

(30) Priority : **30.07.90 JP 204851/90**

(43) Date of publication of application : **05.02.92 Bulletin 92/06**

(84) Designated Contracting States : **DE FR GB NL**

(71) Applicant : **SHARP KABUSHIKI KAISHA**
**22-22 Nagaike-cho Abeno-ku**
**Osaka (JP)**

(72) Inventor : **Shiomi, Makoto**
**2613-1, Ichinomoto-cho**
**Tenri-shi, Nara-ken (JP)**
Inventor : **Koden, Mitsuhiro**
**2-1-30-503, Suzaku 5-chome**
**Nara-shi, Nara-ken (JP)**
Inventor : **Nakagawa, Kenichi**
**D-304, Oonakazato 1414**
**Fujinomiya-shi, Shizuoka-ken (JP)**
Inventor : **Funada, Fumiaki**
**33-10, Izumihara-cho**
**Yamatokoriyama-shi, Nara-ken (JP)**

(74) Representative : **West, Alan Harry et al**
**R.G.C. Jenkins & Co. 26 Caxton Street**
**London SW1H 0RJ (GB)**

(54) **Ferroelectric liquid crystal composition and liquid crystal device comprising the same.**

(57) A ferroelectric liquid crystal composition comprising at least one compound of the formula (I) :

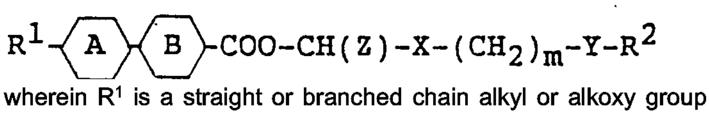
(I)

wherein $R^1$ is a straight or branched chain alkyl or alkoxy group having 1 to 15 carbon atoms

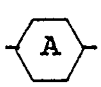 and 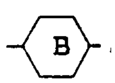

are, the same or different, a phenylene group which may be substituted by a halogen or a cyclohexylene group, X is a single bond or a vinyl or cyclopropylene group, Y is a methylene group or -COO- group, Z is a mono-, di- or trifluoromethyl group, m is an integer of 1 to 15 and $R^2$ is a straight or branched chain alkyl group having 1 to 15 carbon atoms or a phenyl group, and a base liquid crystal material. This composition is used for a ferroelectric liquid crystal device .

EP 0 469 800 A2

The present invention relates to ferroelectric liquid crystal compositions and liquid crystal devices comprising the above compositions.

Liquid crystal display devices most widely used presently are those utilizing nematic phase. However, twisted nematic (TN) liquid crystal devices decrease in contrast with an increase in the number of lines, so that it is practically impossible to fabricate large-capacity liquid crystal devices of this type with 2000 x 2000 lines. To overcome the drawback of TN liquid crystal display devices, supertwisted nematic (STN) liquid crystal display devices and double supertwisted nematic (DSTN) liquid,crystal display devices have been developed, whereas these devices still have the drawback of decreasing in contrast and in the response speed with increasing number of lines. Therefore, such devices are limited to a display capacity of about 800 x 1024 lines. On the other hand, the prior art has already provided liquid crystal display devices of the active matrix type wherein thin film transistors (TFT) are arranged on a substrate. Although it is technically possible to give devices of this type a large display capacity, for example, of 1000 x 1000, these devices have the drawback of necessitating a long production process and being low in yield and therefore very costly to fabricate.

In recent years, studies have largely made about various display modes utilizing smectic phase in addition to liquid crystal display devices utilizing the nematic phase. Particularly, as promising means, ferroelectric liquid crystal display devices are proposed [see N.A. Clark et al., Appl. Phys. Lett., 36, 899(1980)]. The proposed device utilized the chiral smectic C phase, chiral smectic I phase of ferroelectric liquid crystals. The device can be given a great display capacity with an improved response speed since the memory property of the crystals is utilized. Further more, the device can be produced at a low cost since it does not need to use active components such as thin-film transistors. The ferroelectric liquid crystal display device also offers the wider field of view. Thus, the device appears very promising as a large capacity-display device having at least 2000 x 2000 lines.

The liquid crystal materials for use in the ferroelectric liquid crystal display device wherein smectic C phase is utilized must of course exhibit smectic C phase over a wide temperature range around room temperature and needs to fulfill various other requirements.

First, the device for large capacity display must have high speed responsiveness, and from this viewpoint, the liquid crystal materials need to be highly amenable to spontaneous polarization and low in viscosity. Further to obtain satisfactory orientation and bistability when used for the liquid crystal cell, the liquid crystal material needs to exhibit the phase series of IAC (Isotropic-Smectic A-Smectic C), or INAC (Isotropic-Nematic-Smectic A-Smectic C), and the helical pitch of nematic phase and smectic C phase needs to be sufficiently larger than the cell length. It is also desired that the material be great in tilt angle which is relevant to the contrast and brightness of liquid crystal display. Moreover, it is required to optimize dielectric anisotropy, refractive index and specific resistance.

At present, however, it is impossible for a single compound to fulfill all the desired requirements, so that a plurality of compounds are usually mixed together for use as a liquid crystal composition. To prepare a liquid crystal composition fulfilling the requirements for actual use, it is necessary to use numerous single liquid crystal compounds having a wide variety of properties. It is sometimes likely that compounds which per se exhibit no liquid crystal properties will be useful as components of the liquid crystal composition.

Prior art documents concerning the ferroelectric liquid crystal composition include Japanese Laid-Open Patent Publication No. HEI 2(1990)-69440, Japanese Laid-Open Patent Publication No. SHO 63(1988)-307837, British Patent No. 2,216,540 and U.S. Patent No. 5,026,506.

The present invention has been accomplished in view of the above situation, and provides a ferroelectric liquid crystal composition having a wide operation temperature range, excellent orientation, excellent memory property and large tilt angle as well as exhibiting high-speed responsiveness at high temperature, and a liquid crystal display device comprising the above-mentioned composition.

Thus, the present invention provides a ferroelectric liquid crystal composition comprising at least one compound of the following formula (I):

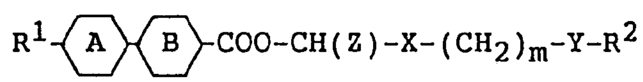 (I)

wherein $R^1$ is a straight or branched chain alkyl or alkoxy group having 1 to 15 carbon atoms,

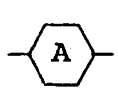 and 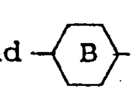

are the same or different, a phenylene group which may be substituted by a halogen or a cyclohexylene group, X is a single bond or a vinyl or cyclopropylene group, Y is a methylene group or -COO- group, Z is a mono- , di- or trifluoromethyl group, m is an integer of 1 to 15 and $R^2$ is a straight or branched chain alkyl group having 1 to 15 carbon atoms or a phenyl group, and a base liquid crystal material. The base liquid crystal material comprises at least one compound of the following formula (II):

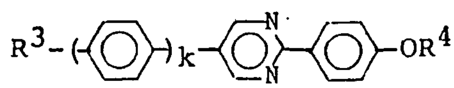

(II)

wherein $R^3$ and $R^4$ are, the same or different, a straight or branched chain alkyl or alkyloxy group having 1 to 15 carbon atoms and k is an integer of 0 or 1 and/or at least one compound of the following formula (III):

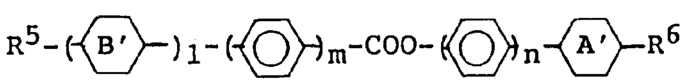

(III)

wherein $R^5$ and $R^6$ are, the same or different, a straight or branched chain alkyl or alkyloxy group having 1 to 15 carbon atom,

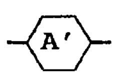

and

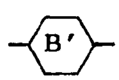

are, the same or different, a phenylene group which may be substituted by a halogen or a cyclohexylene group, 1, m and n each is an integer of 0 or 1 and the sum of 1, m and n is 1, 2 or 3.

The compounds of the formula (I) are believed to be novel ones which have not been disclosed so far in any documents.

The optically active compounds represented by the formula (I) include the isomers of trans-form and cis-form, both of which can be used for the present invention. The aforesaid isomers may be admixed for use.

In the compound (I), the straight or branched chain alkyl group having 1 to 15 carbon atoms include, e.g., methyl, ethyl, propyl, i-propyl, butyl, i-butyl, pentyl, 1- or 2-methylbutyl, hexyl, 1- or 3- methylpentyl, heptyl, 1- or 4-methylhexyl, octyl, 1-methylheptyl, nonyl, 1- or 6-methyloctyl, decyl, 1-methylnonyl, undecyl, 1-methyldecyl, dodecyl, 1-methylundecyl or the like. The branched chain alkyl or alkyloxy groups may contain an asymmetric carbon atom. Further, one or more hydrogen atoms in these alkyl or alkyloxy groups may be substitued with a fluorine atom, chlorine atom, bromine atom, cyano group, nitro group, trifluoromethyl group, methoxy group or the like.

The phenylene group which may be substituted by a halogen with respect to the definition of

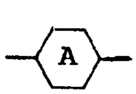 or 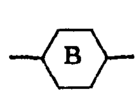

may be phenylene group or monofluorophenylene group.

The compound (I) of the present invention can be prepared, e.g., by reacting the corresponding carboxylic acid with phosphorus pentachloride to convert into the carboxylic acide chloride, and reacting the resulting carboxylic acid chloride with sodium alkoxide as formed by the reaction of the corresponding optically active alcohol and sodium hydride. The reactions can be shown by the following chemical formulae.

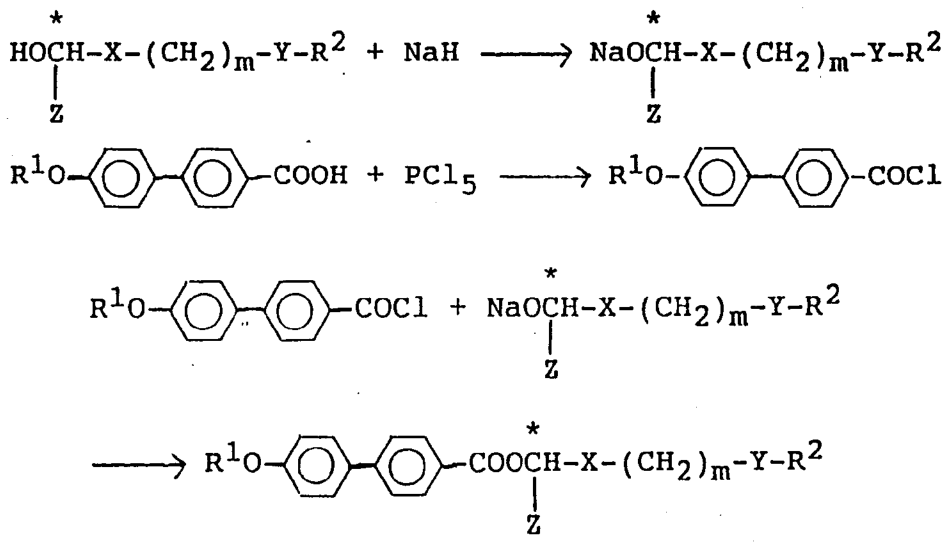

The optically active alcohols to be used in the above reaction can be synthesized according to, e.g., Takashi Tsukamoto, Michinori Takeda, Masazoe Hayashi, Takashi Yamazaki & Tomoya Kitazume, "Fluorine Chemistry Discussion, XIII, Transactions" (1988), 4D, 112.

The compounds (I) do not always exhibit a liquid crystal phase. However, the addition of the compounds (I) in a suitable amount to a nonchiral smectic liquid compound or its composition or chiral smectic liquid compound or its composition increases the spontaneous polarization and high-speed responsiveness. Although any of the optically active compounds (I) wherein Z is a mono- , di- or trifluoro-methyl group can be used, the one in which Z is a trifluoro-methyl group is more preferable.

The ferroelectric liquid crystal composition contains the compound (I) usually in an amount of 0.1 to 30 wt.%, preferably 0.5 to 10 wt.%, more preferably 2.0 to 10 wt.% based on the total weight of the composition. If the amount is over 30 wt.%, there arises practical problems such that the compound added thereto is crystallized in the ferroelectric liquid crystal composition and the upper limit temperature decreases in the smectic C phase, whereas if the amount is less than 0.1 wt.%, fully improved responsiveness will not be available.

In the compound (II), the straight or branched chain alkyl group having I to 15 carbon atoms includes, e.g., methyl, ethyl, propyl, i-propyl, butyl, i-butyl, pentyl, 1-or 2-methylbutyl, hexyl, 1- or 3-methylpentyl, heptyl, 1-or 4-methylhexyl, octyl, 1-methylheptyl, nonyl, 1- or 6-methyloctyl, decyl, 1-methylnonyl, undecyl, 1-methyldecyl, dodecyl, 1-methylundecyl or the like. The branched chain alkyl or alkyloxy groups may contain an asymmetric carbon atom. Further, one or more hydrogen atoms in these alkyl or alkyloxy groups may be substituted with a fluorine atom, chlorine atom, bromine atom, cyano group, nitro group, trifluoromethyl group, methoxy group or the like.

The compound (II) can be prepared, e.g., by the method disclosed in Zaschke, H,; Schubert, H.; Kuschel, F.; Dinger, F. and Demus, D.; DDR WP 95892.

When the compound (II) is used as a base liquid crystal material, it can exhibit stable smectic C phase, and hence can produce a ferroelectric liquid crystal composition where a temperature range in the chiral smectic C phase is wide and responsive speed is high. Usually, the compounds (II) are used in a mixture thereof. The compound (II) occupies usually in the range of 30 to 99%, preferably 50 to 85% of the composition.

In the compound (III), the straight or branched chain alkyl group having 1 to 15 carbon atoms includes, e.g., methyl, ethyl, propyl, i-propyl, butyl, i-butyl, pentyl, 1-or 2-methylbutyl, hexyl, 1- or 3-methylpentyl, heptyl, 1-or 4-methylhexyl, octyl, 1-methylheptyl, nonyl, 1- or 6-methyloctyl, decyl, 1-methylnonyl, undecyl, 1-methyldecyl, dodecyl, 1-methylundecyl or the like. The branched chain alkyl or alkyloxy groups may contain an asymmetric carbon atom. Further, one or more hydrogen atoms in these alkyl or alkyloxy groups may be substituted with a fluorine atom, chlorine atom, bromine atom, cyano group, nitro group, trifluoromethyl group, methoxy group or the like.

The phenylene group which may be substituted by a halogen as defined in the definition of

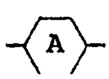 or 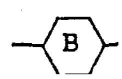

is also applicable to that of

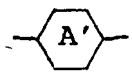 or 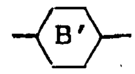

of the compound (III).

The compound (III) can be prepared, e.g., by the method disclosed in Gray, G.W.; Goodby. J. W.; Mol. Cryst. Liq. Cryst. 37, 157, (1976).

When the compound (III) is used as a base liquid crystal material, it is effective for increasing the upper-limit temperature of chiral smectic C phase, widening the temperature range of the nematic phase and decreasing the temperature for crystallization. The compounds (III) are usually used in a mixture thereof. The compounds (III) to be contained are usually 5 to 99 wt.%, preferably 10 to 45 wt.% of the composition. If the concentration of the compound (III) is low, a satisfactory effect cannot be obtained, while high concentration causes unfavorable phenomena such as crystallization at low temperature, increase in viscosity or the like. Accordingly, the combination use of the compounds (III) and (II) is preferable.

The ferroelectric liquid crystal composition may be prepared by adding, in a suitable amount, compounds other than the compounds (I), (II) and (III) such as:

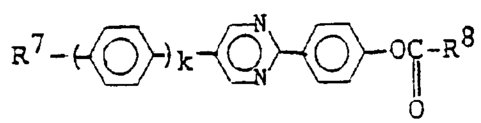

wherein $R^7$ and $R^8$ have the same meanings as defined for $R^5$ and $R^6$ and k is the same as defined above, or

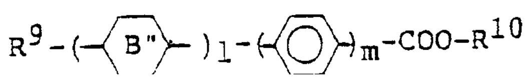

wherein $R^9$ and $R^{10}$ have the same meanings as defined for $R^5$ and $R^6$ and 1 and m have the same meanings as defined above, or

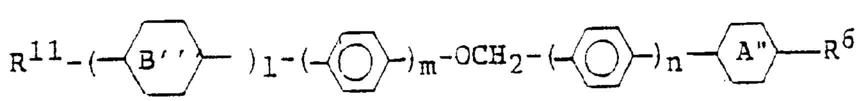

wherein $R^{11}$ and $R^{12}$ have the same meanings as defined for $R^5$ and $R^6$,

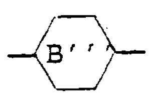

and

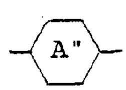

have the same meanings as defined for

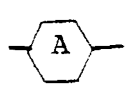

and

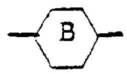

and 1, m and n are the same as defined above.

The amount of these compounds to be contained is usually less than 40 wt.% of the composition.

The compounds (I) increase the spontaneous polarization and responsive speed of the ferroelectric liquid crystal composition. The compounds (II) widen the temperature range of the ferroelectric liquid crystal composition showing chiral smectic C phase and decrease viscosity. The compounds (III) widen the temperature range of the ferroelectric liquid crystal composition showing chiral smectic C phase and nematic phase and increase the tilt angle.

The ferroelectric liquid crystal composition having various excellent characteristics can be prepared by mixing these compounds at a suitable ratio.

The present invention further provides a liquid crystal device which comprises the ferroelectric liquid crystal composition disposed between a pair of electrodes. With the exception of using the ferroelectric liquid crystal composition of the invention described above, the device can be of known construction and fabricated with use of materials already known in the art. The cell length is preferably 0.8 to 10.0 μm, more preferably 1.2 to 6.0 μm. The liquid crystal device, which is useful as a display device, is also advantageously usable as a liquid crystal shutter.

The present invention will be described in greater detail with reference to the following examples, which, nevertheless, in no way limit the invention.

Preparation 1 (Compound No. 4)

Preparation of (R)-4'-n-octyloxy-bipheny-4-carboxylic acid 2,2,2-trifluoro-1-(2-hexyl-(1S,2R)-(trans) cyclopropyl)ethyl ester

Phosphorus pentachloride (0.4 g, 1.9 m mol) was added to 4'-n-octyloxy-biphenyl-4-carboxylic acid (0.5 g, 1.5 m mol), followed by heating. The reaction mixture was distilled under reduced pressure to remove phosphorus oxychloride and an excess of phosphorus pentachloride, affording 4'-n-octyloxy-biphenyl-4-carboxylic acid chloride. Subsequently, (R)-2,2,2-trifluoro-1-(2-hexyl-(1S,2R)-(trans)-cyclopropyl) ethanol (0.3 g, 1.5 m mol) was dissolved in toluene (10 ml), to which sodium hydride (0.05 g, 2m mol) was added. Unreacted sodium hydride was filtered off to obtain sodium (R)-2,2,2-trifluoro-1-(2-hexyl-(1S,2R)-(trans)-cyclopropyl) ethoxide. The above acid chloride dissolved in toluene (10 ml) was added to the resultant compound. The mixture was allowed to stand for 12 hours at room temperature, maintained for 3 hours at 80°C and cooled. This reaction mixture was then added to distilled water and extracted with ethyl ether. The ether layer was washed with water, dried over $Na_2 SO_4$ and distilled to remove the solvent. The residue was purified by a liquid chromatography (column: C18-silica, mobile phase solvent: methanol) to give the title compound (Compound No. 4).

Preparation 2 (Compound No. 2)

Preparation of (S)-4'-n-octyloxy-biphenyl-4-carboxylic acid 1-trifluoromethyl-(2-ethoxycarbonyl) ethyl ester

Phosphorus pentachloride (0.4 g, 1.9 m mol) was added to 4'-n-octyloxy-biphenyl-4-carboxylic acid (0.5 g, 1.5 m mol), followed by heating. The reaction mixture was distilled under reduced pressure to remove phosphorus oxychloride and an excess of phosphorus pentachloride, affording 4'-n-octyloxy-biphenyl-4-carboxylic acid chloride. The obtained compound was dissolved in pyridine, to which (S)-1-trifluoromethyl-(2-carboxylic acid ethyl)-ethanol (0.3 g, 1.5 m mol) was added. The mixture was allowed to stand for 12 hours at room temperature, maintained for 3 hours at 80°C and cooled. This reaction mixture was then added to an aqueous hydrogen chloride solution and extracted with diethyl ether. The ether layer was washed with $NaHCO_3$ solution and water successively, dried over $Na_2SO_4$ and distilled to remove the solvent. The residue was purified by a column chromatography (solvent: chloroform) to give the title compound (Compound No. 2).

Preparation 3 (Compound No. 5)

Preparation of (S)-4'-n-octyloxy-biphenyl-4-carboxylic acid 1-trifluoromethyl-3-phenyl (trans)-2-propenyl ester

Phosphorus pentachloride (0.4 g, 1.9 m mol) was added to 4'-n-octyloxy-biphenyl-4-carboxylic acid (0.5

g, 1.5 m mol), followed by heating. The reaction mixture was distilled under reduced pressure to remove phosphorus oxychloride and an excess of phosphorus pentachloride, affording 4'-n-octyloxy-biphenyl-4-carboxylic acid chloride. Subsequently, (S)-1-trifluoromethyl-3-phenyl-(trans)-2-propenylalcohol (0.3 g, 1.5 m mol) was dissolved in toluene (10 ml), to which sodium hydride (0.05 g, 2 m mol) was added. Unreacted sodium hydride was filtered off to obtain (S)-1-trifluoromethyl-3-phenyl-(trans)-2-propenylalkoxide. Acid chloride dissolved in toluene (10 ml) was added to the resultant compound. The mixture was allowed to stand for 12 hours at room temperature, maintained for 3 hours at 80°C and cooled. This reaction mixture was then added to distilled water and extracted with ethyl ether. The ether layer was washed with water, dried over $Na_2SO_4$ and distilled to remove the solvent. The residue was purified by a liquid chromatography (column: C18-silica, mobile phase solvent: methanol) to give the title compound (Compound No. 5).

Preparation 4 (Compound No. 6)

Preparation of (R)-4'-n-octyloxy-biphenyl-4-carboxylic acid 1-trifluoromethyl-3-phenyl-(cis)-2-propenyl ester

The title compound (Compound No. 6) was prepared by the same manner as in Preparation 3 except for using (R)-1-trifluoromethyl-3-phenyl-(cis)-2-propenylalcohol (0.3 g, 1.5 m mol) instead of (S)-1-trifluoromethyl-3-phenyl-(trans)-2-propenylalcohol (0.3 g, 1.5 m mol).

Preparation 5 (Compound No. 3)

Preparation of (S)-4'-n-octyloxy-biphenyl-4-carboxylic acid 1-trifluoromethyl-3-phenyl-propyl ester

Phosphorus pentachloride (0.4 g, 1.9 m mol) was added to 4'-n-octyloxy-biphenyl-4-carboxylic acid (0.5 g, 1.5 m mol), followed by heating. The reaction mixture was distilled under reduced pressure to remove phosphorus oxychloride and an excess of phosphorus pentachloride, affording 4'-n-octyloxy-biphenyl-4-carboxylic acid chloride. Subsequently, (S)-1-trifluoromethyl-3-phenyl-propylalcohol (0.3 g, 1.5 m mol) was dissolved in toluene (10ml), to which sodium hydride (0.05 g, 2 m mol) was added. Unreacted sodium hydride was filtered off to obtain (S)-1-trifluoromethyl-3-phenyl-propylalkoxide. The above acid chloride dissolved in toluene (10 ml) was added to the resultant compound. The mixture was allowed to stand for 12 hours at room temperature, maintained for 3 hours at 80°C and cooled. This reaction mixture was then added to distilled water and extracted with ethyl ether. The ether layer was washed with water, dried over $Na_2SO_4$ and distilled to remove the solvent. The residue was purified by a liquid chromatography (column: C18-silica, mobile phase solvent: methanol) to give the title compound (Compound No. 3).

Preparation 6 (Compound No. 1)

Preparation of (S)-4-(trans-4'-n-penty-cyclohexyl)-2-fluoro-benzoic acid 1-trifluoromethyl-nonyl ester

Phosphorus pentachloride (0.4 g, 1.9 m mol) was added to 4-(trans-4'-n-pentyl-cyclohexyl)-2-fluoro-benzoic acid (0.5 g, 1.5 m mol), followed by heating. The reaction mixture was distilled under reduced pressure to remove phosphorus oxychloride and an excess of phosphorus pentachloride, affording 4-(trans-4'-n-pentyl-cyclohexyl)-2-fluoro-benzoic acid chloride. Subsequently, (S)-1-trifluoromethyl-nonylalcohol (0.3 g, 1.5 m mol) was dissolved in toluene (10 ml), to which sodium hydride (0.05 g, 2 m mol) was added. Unreacted sodium hydride was filtered off to obtain sodium (S)-1-trifluoromethyl-nonylalkoxide. The above acid chloride dissolved in toluene (10 ml) was added to the resultant compound. The mixture was allowed to stand for 12 hours at room temperature, maintained for 3 hours at 80°C and cooled. This reaction mixture was then added to distilled water and extracted with ethyl ether. The ether layer was washed with water, dried over $Na_2SO_4$ and distilled to remove the solvent. The residue was purified by a liquid chromatography (column: C18-silica, mobile phase solvent: methanol) to give the title compound (Compound No. 1).

Example 1

Liquid crystal compositions No. 7 to No. 9 were prepared, each of which has compositions shown in Table 2 and exhibits nonchiral smectic C phase. Ferroelectric liquid crystal display devices Nos. 10 to 19 shown in Table 3 were prepared by adding liquid crystal compositions Nos. 1 to 6 shown in Table 1 in an amount of 2 to 10 wt.% respectively to each of liquid crystal compositions Nos. 7 to 9.

Two glass substrates each was laminated by ITO film electrode, $Si0_2$ insulating film and PI film or PVA film in this order. The uppermost layer was rubbed to make orientation layer. These two substrates were set to make a cell facing their orientation layers to each other, and leaving 1 to 3 μm clearance between these two layers.

Liquid crystal compositions shown in Table 3 were injected respectively to the cell. The cell was then heated to a temperature to change the liquid crystal composition into an isotropic liquid and then cooled to appear smectic phase at 1°C/min, whereby ferroelectric liquid crystal display devices exhibiting good orientation were obtained. Table 3 shows characteristics of the obtained ferroelectric liquid crystal display devices. The response speed was represented by the time required for changing the transmitted light amount in the ratio of 50% when rectangle wave of $V_{p\text{-}p}$ = 10V/µm was applied to the cell.

As apparent from Table 3, high-speed responsiveness was obtained. Since these ferroelectric liquid crystal composition include optically active compounds in a concentration of 2 to 10 wt.% which is relatively low, a helical pitch of nematic phase was larger than the cell length. Therefore, the ferroelectric liquid crystal compositions exhibited excellent orientation.

These ferroelectric liquid crystal compositions, which exhibit good orientation and high-speed responsiveness, can be applied to a ferroelectric liquid crystal display device with a large capacity.

Table 1 Chiral Compounds

Compound No. — Chemical Formulae

1 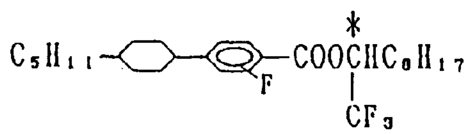

2 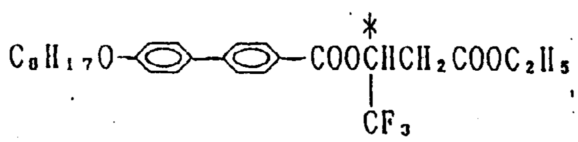

3 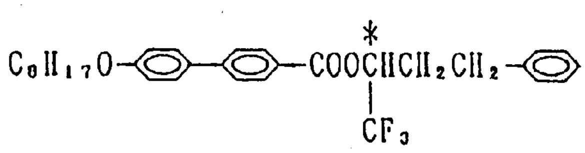

4 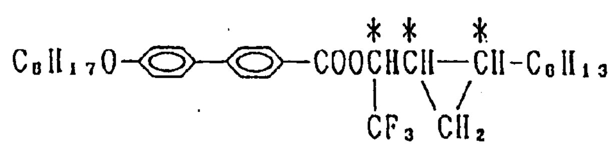

5 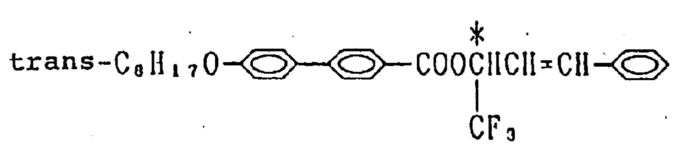

6 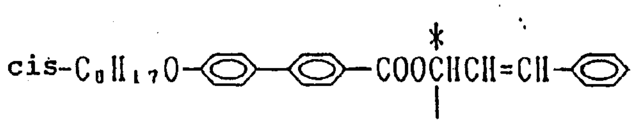

Table 2 Nonchiral Compositions (wt. %)

| Compositions | No.7 | No.8 | No.9 |
| --- | --- | --- | --- |
| 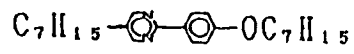 | 5.0 | | |
| 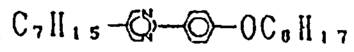 | 10.0 | | |
| 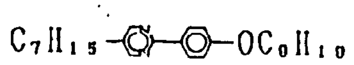 | 15.0 | | 9.9 |
| 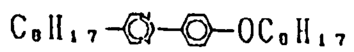 | 20.0 | | 19.3 |
| 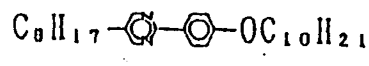 | 30.0 | | 19.5 |
| 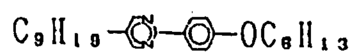 | 20.0 | | 10.6 |
| 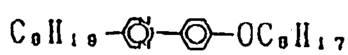 | | | 9.8 |
| 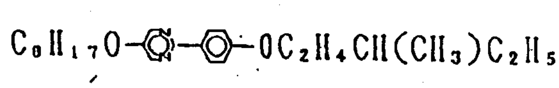 | | | 2.7 |
| 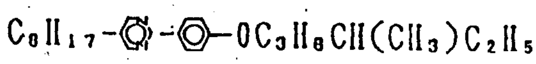 | | | 1.6 |
| 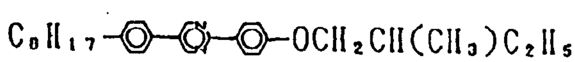 | | | 1.9 |
| 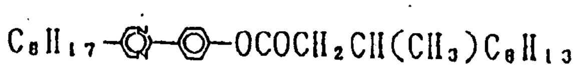 | | | 2.0 |
| 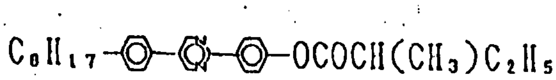 | | | 2.3 |
| 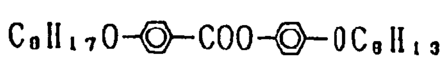 | | 8.1 | |
| 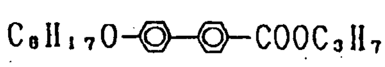 | | | 2.3 |
| 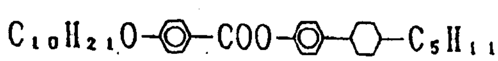 | | 8.0 | 4.0 |
| 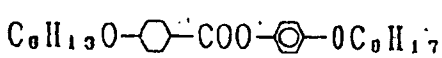 | | 12.2 | 2.0 |

Table 2 (continued)

| Compositions | No.8 | No.9 |
|---|---|---|
| 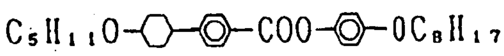 | 3.8 | |
| 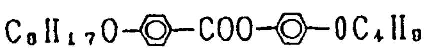 | 7.8 | 2.0 |
| 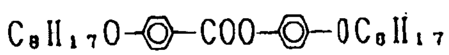 | 8.2 | |
| 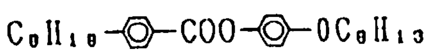 | 11.7 | |
| 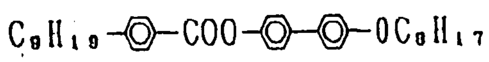 | 3.8 | |
| 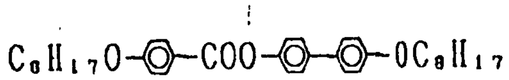 | 2.3 | |
| 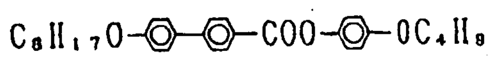 | 2.2 | |
| 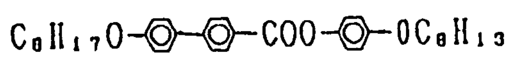 | 1.8 | |
| 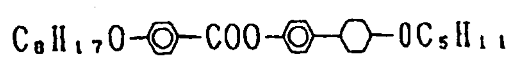 | 8.0 | |
| 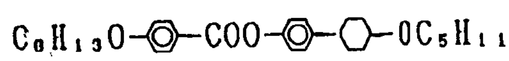 | 4.0 | |
| 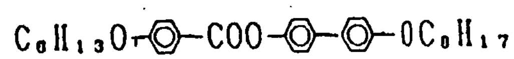 | 1.9 | |
| 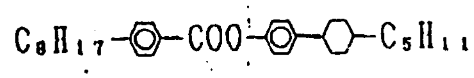 | 9.7 | |
| 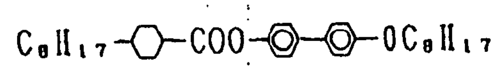 | 2.6 | |
| 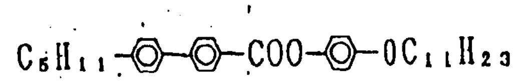 | 3.9 | |
| 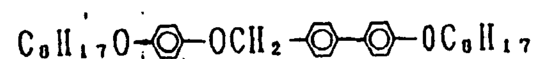 | | 1.9 |
| 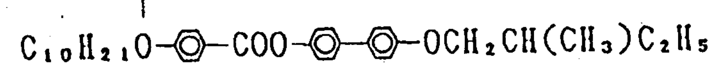 | | 2.1 |
| 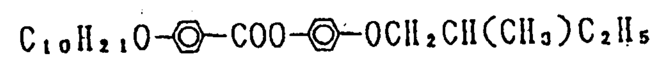 | | 1.9 |
| 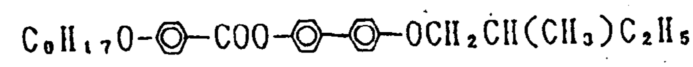 | | 2.3 |
| 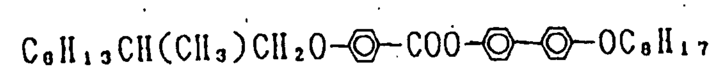 | | 1.9 |

TABLE 3

| Composition No. | Chiral Compound | Chiral conc. (%) | No. 7 conc. (%) | No. 8 conc. (%) | No. 9 conc. (%) | Transition tem. (°C) I·N·A·C | Orientation Film | Cell Gap (μm) | Measuring tem. | Tilt Angle (°) | Response Speed (μsec.) |
|---|---|---|---|---|---|---|---|---|---|---|---|
| 10 | 1 | 2.00 | 98.00 | | | 69·65·45 | PVA | 2.0 | 25 | 11 | 204 |
| 11 | 2 | 2.00 | 98.00 | | | 70·60·45 | PVA | 2.0 | 25 | 13 | 260 |
| 12 | 3 | 2.00 | 98.00 | | | 69·61·44 | PVA | 2.0 | 25 | 30 | 390 |
| 13 | 4 | 2.00 | 98.00 | | | 70·68·41 | PVA | 2.0 | 25 | 19 | 310 |
| 14 | 5 | 2.00 | 98.00 | | | 70·68·60 | PVA | 2.0 | 25 | 21 | 190 |
| 15 | 6 | 2.00 | 98.00 | | | 67·63·43 | PVA | 2.0 | 25 | 21 | 340 |
| 16 | 5 | 5.00 | 95.00 | | | 65·63·58 | PI | 1.3 | 30 | 12 | 46 |
| 17 | 2 | 3.00 | | 97.00 | | 120 ·78 | PI | 1.4 | 50 | 22 | 300 |
| 18 | 4 | 6.00 | | 94.00 | | 115 ·81 | PI | 1.4 | 50 | 15 | 140 |
| 19 | 4 | 3.00 | | | 97.00 | 70·69·56 | PI | 2.5 | 30 | 23 | 230 |

## Claims

1. A ferroelectric liquid crystal composition comprising at least one compound of the formula (I):

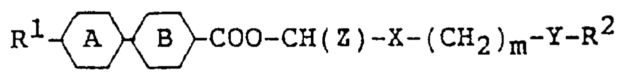

(I)

wherein $R^1$ is a straight or branched chain alkyl or alkoxy group having 1 to 15 carbon atoms,

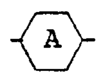 and 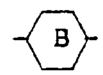

are, the same or different, a phenylene group which may be substituted by a halogen or a cyclohexylene group, X is a single bond or a vinyl or cyclopropylene group, y is a methylene group or -COO- group, Z is a mono-, di- or trifluoromethyl group, m is an integer of 1 to 15 and $R^2$ is a straight or branched chain alkyl group having 1to 15 carbon atoms or a phenyl group, and a base liquid crystal material.

2. A composition of claim 1 in which the compound of the formula (I) is contained in an amount of 0.1 to 30 % by weight based on the total weight of the composition.

3. A composition of claim 1 in which the compound of the formula (I) is contained in an amount of 2 to 10 % by weight based on the total weight of the composition.

4. A composition of claim 1 in which $R^1$ is a $C_{5-8}$ alkyl or $C_{5-8}$ alkoxy group, Z is trifluoromethyl group, and $R^2$ is a $C_{2-8}$ alkyl group or a phenyl group.

5. A composition of claim 1 in which the compound (I) is (S)-4-(trans-4'-n-pentyl-cyclohexyl)-2-fluoro-benzoic acid 1-trifluoromethyl-nonyl ester, (S)-4'-n-octyloxy-biphenyl-4-carboxylic acid 1-trifluoromethyl-(2-ethoxycarbonyl)ethyl ester, (S)-4'-n-octyloxy-biphenyl-4-carboxylic acid 1-trifluoromethyl-3-phenyl-propyl ester, (R)-4'-n-octyloxy-biphenyl-4-carboxylic acid 2,2,2-trifluoro-1-(2-hexyl-(1S,2R)-(trans)-cyclopropyl)ethyl ester, (S)-4'-n-octyloxy-biphenyl-4-carboxylic acid 1-trifluoromethyl-3-phenyl-(trans)-2-propenyl ester, (R)-4'-n-octyloxy-biphenyl-4-carboxylic acid 1-trifluoromethyl-3-phenyl-(cis)-2-propenyl ester.

6. A composition of claim 1 in which the base liquid crystal material comprises at least one compound of the formula (II):

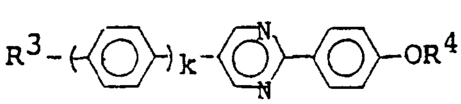

(II)

wherein $R^3$ and $R^4$ are, the same or different, a straight or branched chain alkyl or alkoxy group having 1 to 15 carbon atoms and k is an integer of 1 or 2.

7. A composition of claim 1 in which the base liquid crystal material comprises at least one compound of the formula (III):

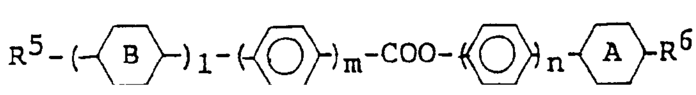

(III)

(wherein $R^5$ and $R^6$ are, the same or different, a straight or branched chain alkyl or alkyloxy group having 1 to 15 carbon atoms,

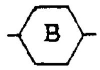 and 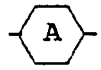

are, the same or different, a phenylene group which may be substituted by a halogen or a cyclohexylene group, 1, m and n each is an integer of 0 or 1 and the sum of 1, m and n is 1, 2 or 3.

**8.** A composition of claim 7 in which the base liquid crystal material comprises at least one compound (II) and at least one compound (III).

**9.** A liquid crystal device comprising a pair of substrates, an orientation controlling layer provided on at least one of said substrates and a ferroelectric liquid crystal layer provided between said pair of substrates, said ferroelectric liquid crystal layer comprising the ferroelectric liquid crystal composition as claimed in any one of claims 1 to 8.